# EUROPEAN PATENT APPLICATION

(11) **EP 2 878 251 A1**
(43) Date of publication of application: **03.06.2015**
(21) Application number: 13822434.0
(22) Date of filing: 02.07.2013
(51) Int. Cl.: A61B 1/00, A61B 5/07, H02J 17/00

(54) **BIOLOGICAL INFORMATION ACQUISITION SYSTEM**

(30) Priority: 27.07.2012 JP 2012167243
(71) Applicant: Olympus Corporation, Shibuya-ku Tokyo 151-0072 (JP)
(72) Inventor: YASUNAGA, Shinji, Tokyo 151 0072 (JP)
(74) Representative: Winter, Brandl, Fürniss, Hübner, Röss, Kaiser, Polte - Partnerschaft mbB
(86) International application number: PCT/JP2013/068104
(87) International publication number: WO 2014/017259

(57) **Abstract**

Provided is a living body information acquisition system including a living body information acquisition apparatus configured to acquire living body information of an inside of a subject, and a power supply apparatus configured to be able to wirelessly supply power necessary for driving each part of the living body information acquisition apparatus, in which the living body information acquisition apparatus has a power receiving section capable of receiving the power that is supplied by the power supply apparatus, and an extension part that is provided so as to extend from the main body part of the living body information acquisition apparatus, and is formed from a flexible member which can contain thereinside at least a part of the power receiving section.

## Description

### Technical Field

The present invention relates to a living body information acquisition system, and particularly to a living body information acquisition system performing wireless power supply.

### Background Art

An endoscope in a medical field has been conventionally used in applications such as for observing inside a living body. Further, as one type of the aforementioned endoscope, a capsule endoscope has been recently proposed, which is disposed in a body cavity by being swallowed by a subject, acquires an image of an object while moving through the body cavity in accordance with peristaltic movement, and can transmit the acquired image of the object to the outside as a radio signal.

For example, Japanese Patent Application Laid-Open Publication No. 2006-280829 discloses a system configured to include a capsule endoscope as described above. Specifically, Japanese Patent Application Laid-Open Publication No. 2006-280829 discloses a configuration of an endoscope system having a capsule endoscope and an extracorporeal unit, in which wireless power supply from the extracorporeal unit to the capsule endoscope is performed by utilizing electromagnetic induction phenomenon.

However, the configuration disclosed in Japanese Patent Application Laid-Open Publication No. 2006-280829 has a problem in that the positional relationship between a power transmission antenna provided in the extracorporeal unit and a power receiving antenna provided in the capsule endoscope inevitably imposes a relatively large effect on transmission efficiency of power. As a result, according to the configuration disclosed in Japanese Patent Application Laid-Open Publication No. 2006-280829, for example, depending on both orientations of the power transmission antenna and the power receiving antenna, the transmission efficiency of the power supplied from the extracorporeal unit to the capsule endoscope may not reach a predetermined transmission efficiency, unavoidably resulting in a situation in which sufficient power for operations relating to acquisition of images by the capsule endoscope is not supplied.

The present invention has been made in view of the above described circumstances, and has its object to provide a living body information acquisition system which can prevent, as much as possible, deterioration of the transmission efficiency of power in wireless power supply.

### Disclosure of Invention

### Means for Solving the Problem

A living body information acquisition system according to one aspect of the present invention is a living body information acquisition system comprising a living body information acquisition apparatus configured to acquire living body information of an inside of a subject, and a power supply apparatus configured to be able to wirelessly supply power necessary for driving each part of the living body information acquisition apparatus, wherein the living body information acquisition apparatus has a power receiving section that is capable of receiving power supplied from the power supply apparatus, and an extension part that is provided so as to extend from a main body part of the living body information acquisition apparatus, and is formed from a flexible member which can contain thereinside at least a part of the power receiving section.

### Brief Description of the Drawings

Fig. 1 is a diagram showing a configuration of a principal part of a living body information acquisition system relating to an embodiment.
Fig. 2 is a diagram showing an example of an external shape before use of a capsule endoscope relating to the embodiment.
Fig. 3 is a diagram showing an example of an external shape during use of the capsule endoscope relating to the embodiment.
Fig. 4 is a flowchart showing an example of control to be carried out in a living body information acquisition system relating to the embodiment.
Fig. 5 is a diagram showing an example of an external shape during use of a capsule endoscope relating to a variant of the embodiment.

### Best Mode for Carrying Out the Invention

Hereinafter, an embodiment of the present invention will be described with reference to the drawings.

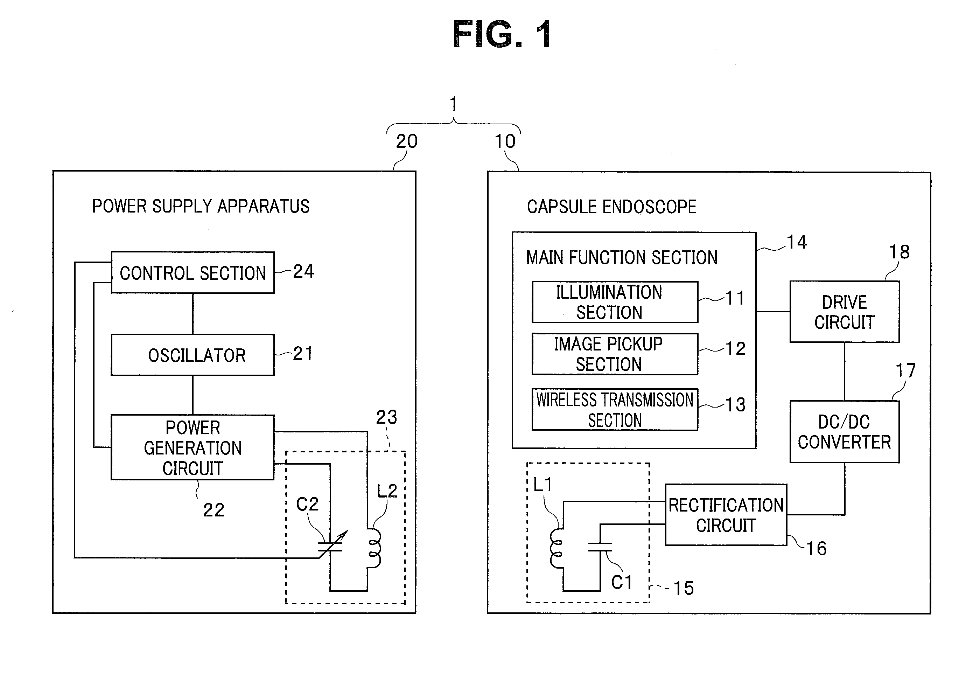

Figs. 1 to 5 relate to the embodiment of the present invention. Fig. 1 is a diagram showing a configuration of a principal part of a living body information acquisition system according to the embodiment.

As shown in Fig. 1, a living body information acquisition system 1 is configured to include a capsule endoscope 10 which has a size and a shape etc. so as to be able to be disposed in a body cavity of a subject, and a power supply apparatus 20 which is disposed outside the capsule endoscope 10 and configured to be able to wirelessly supply power necessary for driving each part of the capsule endoscope 10.

The capsule endoscope 10 is configured to have functions as a living body information acquisition apparatus. Specifically, as shown in Fig. 1, the capsule endoscope 10 has a main function section 14 including: an illumination section 11 having a light emitting device such as an LED that emits illumination light for illuminating an object in a body cavity of a subject; an image pickup section 12 for picking up an image of the object illuminated by the illumination section 11 to acquire image data; and a wireless transmission section 13 for modulating the image data acquired by the image pickup section 12 into a radio signal to transmit the radio signal to the outside.

Moreover, as shown in Fig. 1, the capsule endoscope 10 includes: a power receiving circuit 15 configured to be able to receive alternating current power in accordance with an alternating magnetic field emitted from the power supply apparatus 20; a rectification circuit 16 for converting the alternating current power received at the power receiving circuit 15 into direct current power and outputting the same; a DC/DC converter 17 for converting a voltage level of direct current voltage included in the direct current power to be inputted via the rectification circuit 16 into a voltage level suitable for operation of the main function section 14 to output the direct current voltage; and a driving circuit 18 for driving each part of the main function section 14 by using the direct current power to be inputted via the DC/DC converter 17.

As shown in Fig. 1, the power receiving circuit 15 is configured to have a power receiving coil L1, and a power receiving capacitor C1.

Specifically, according to the power receiving circuit 15 of Fig. 1, one end part of the power receiving coil L1 is connected to the rectification circuit 16, and the other end part of the power receiving coil L1 is connected to one end part of the power receiving capacitor C1. Further, according to the power receiving circuit 15 of Fig. 1, the other end part of the power receiving capacitor C1 is connected to the rectification circuit 16.

That is, the power receiving circuit 15 of the present embodiment is configured as a series resonant circuit that resonates at a series resonance frequency defined by inductance of the power receiving coil L1 and capacitance of the power receiving capacitor C1.

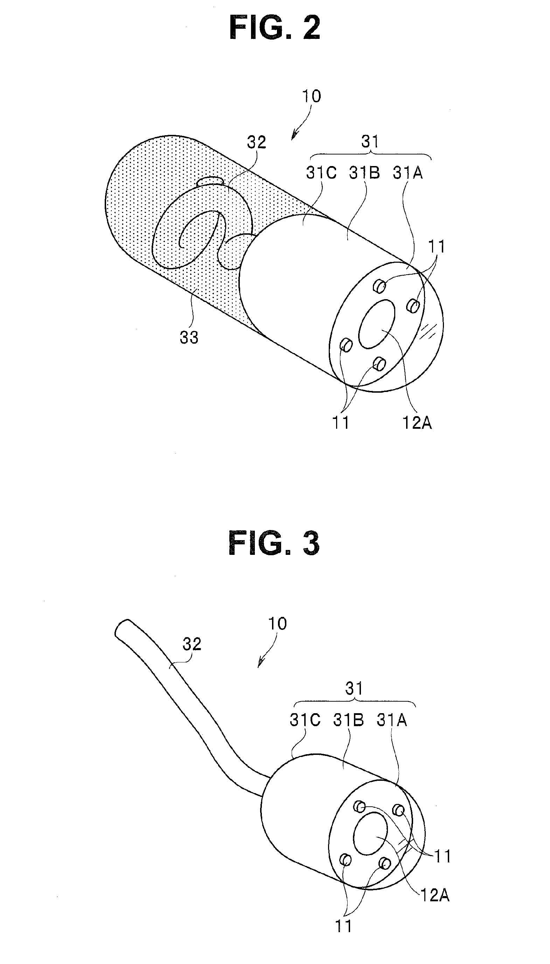

On one hand, the capsule endoscope 10 of the present embodiment is formed to have an external shape, for example, as shown in Figs. 2 and 3. Fig. 2 is a diagram showing one aspect of an external shape before use of the capsule endoscope included in the living body information acquisition system relating to the embodiment. Fig. 3 is a diagram showing one aspect of the external shape during use of the capsule endoscope included in the living body information acquisition system relating to the embodiment.

Specifically, as shown in Figs. 2 and 3, the capsule endoscope 10 is configured to have a main body part 31 configured as a housing having a capsule shape, and an extension part 32 extending from an end part 31C of the main body part 31.

An end part 31A of the main body part 31 is formed from a transparent member having a dome shape, and is configured to be able to transmit illumination light emitted from the illumination section 11, and return light incident on an objective lens 12A of an image pickup section 12, respectively. The cylindrical part 31B of the main body part 31 is formed from a light shielding member having a cylindrical shape. An end part 31C of the main body part 31 is formed from a light shielding member having a dome shape.

The extension part 32 is formed from a flexible member such as of resin. Further, for example, the extension part 32 is configured such that copper wire of a spiral shape which constitutes at least a part of a power receiving coil L1 of a power receiving circuit 15 is embedded inside the flexible member such as of resin. Then, according to such configurations of the extension part 32 and the power receiving coil L1, since at least a part of the power receiving coil L1 is disposed outside the main body part 31 of the capsule endoscope 10, it is possible to reduce the size of the main body part 31 of the capsule endoscope 10.

Further, as shown in Fig. 2, before the capsule endoscope 10 is actually used (before it is displaced in a body cavity of a subject), the extension part 32 is provided in a state of being folded into a predetermined shape and covered with a covering part 33.

The covering part 33 is formed of a water soluble substance, such as a sugar coating, that readily dissolves when the capsule endoscope 10 is actually used (when disposed in a body cavity of a subject). Further, the covering part 33 is formed into a shape, such as a dome shape, which is less likely to cause difficulty in swallowing the capsule endoscope 10.

Then, according to the configurations of the extension part 32 and the covering part 33 as described above, before the capsule endoscope 10 is actually used, for example, the extension part 32 is fixed in a state of being folded into a predetermined shape due to the presence of the covering part 33 as shown in Fig. 2. Further, according to the configuration of the extension part 32 and the covering part 33 as described so far, resulting from that the covering part 33 dissolves (disappears) when the capsule endoscope 10 is actually used, the extension part 32 is deformed (bent) according to the movement of the capsule endoscope 10 for example as shown in Fig. 3.

On the other hand, as shown in Fig. 1, the power supply apparatus 20 includes: an oscillator 21; a power generation circuit 22 for generating a power signal based on a signal outputted from the oscillator 21; a power transmission circuit 23 for generating an alternating magnetic field in accordance with the power signal generated by the power generation circuit 22; and a control section 24 for performing various control.

The power transmission circuit 23 is configured to include a power transmission coil L2 and a power transmission capacitor C2 as shown in Fig. 1.

Specifically, according to the power transmission circuit 23 of Fig. 1, one end part of the power transmission coil L2 is connected to the power generation circuit 22, and the other end part of the power transmission coil L2 is connected to one end part of the power transmission capacitor C2. Further, according to the power transmission circuit 23 in Fig. 1, the other end part of the power transmission capacitor C2 is connected to the power generation circuit 22. Further, according to the power transmission circuit 23 of Fig. 1, the capacitance of the power transmission capacitor C2 is configured to be changeable according to control in the control section 24.

That is, the power transmission circuit 23 of the present embodiment is configured as a series resonant circuit that resonates at a series resonance frequency defined by inductance of the power transmission coil L2 and capacitance of the power transmission capacitor C2. Further, according to the power transmission circuit 23 of the present embodiment, configuration is made such that the series resonant frequency varies as the capacitance of the power transmission capacitor C2 is changed according to control in the control section 24.

The control section 24 is configured to be able to perform control to change the capacitance of the power transmission capacitor C2. Moreover, the control section 24 is configured to be able to measure a reflection level indicating the magnitude of the power signal reflected from the power transmission circuit 23 to the power generation circuit 22. The control section 24 is also configured to be able to measure a phase difference between the current and the voltage (based on the current and voltage waveforms) in the power signal reflected from the power transmission circuit 23 to the power generation circuit 22. Further, the control section 24 is configured to be able to perform control to change the oscillation frequency of the oscillator 21. Note that the details of control in the control section 24 will be described later.

Next, operation and others of the living body information acquisition system 1 relating to the present embodiment will be described.

First, the power source of the power supply apparatus 20 is turned on, the capsule endoscope 10 in which the extension part 32 is covered with the covering part 33 and is fixed (see Fig. 2) is taken out from a container not shown, and further the taken out capsule endoscope 10 is disposed in a body cavity of a subject.

After that, the covering part 33 dissolves as body fluid present in the body cavity of the subject adheres thereto, resulting in a state where the extension part 32 is deformable (bendable) according to the movement of the capsule endoscope 10 (see Fig. 3).

Then, in a state where the extension part 32 is deformable (bendable) according to the movement of the capsule endoscope 10, it is possible to prevent, as much as possible, deterioration of the transmission efficiency of power, which is caused depending on both orientations of the power receiving coil L1 and the power transmission coil L2.

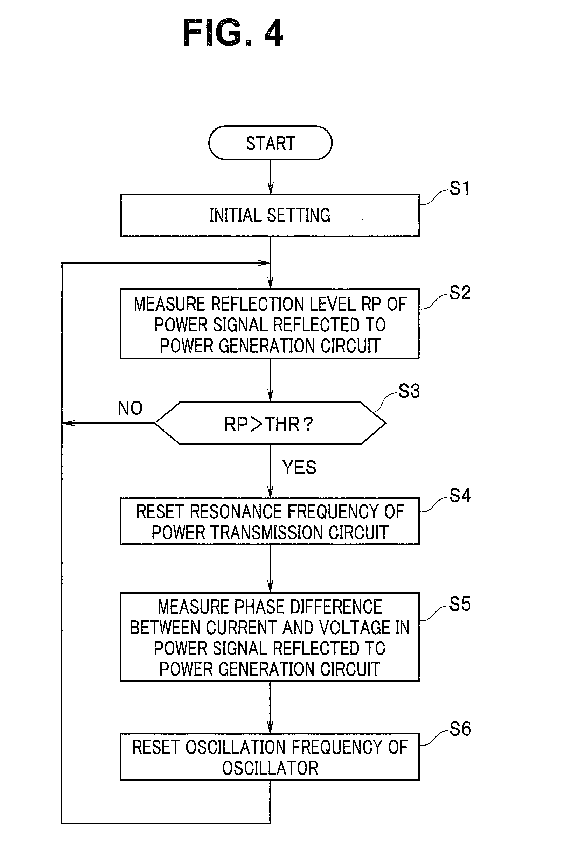

On the other hand, due to variation of the inductance of the power receiving coil L1 according to deformation of the extension part 32, the series resonant frequency of the power receiving circuit 15 may also vary. Therefore, in the present embodiment, in order to maintain the transmission efficiency of power at a high efficiency in wireless power supply, for example, control to change the series resonance frequency of the power transmission circuit 23 following the variation of the series resonance frequency of the power receiving circuit 15 is performed in the control section 24. A specific example of such control will be described below with reference to Fig. 4. Fig. 4 is a flowchart showing an example of control carried out in a living body information acquisition system according to the embodiment.

First, after the power source of the power supply apparatus 20 is turned on, the control section 24 performs initial setting based on data stored in a memory (not shown) or the like (step S1 of Fig. 4).

Specifically, the control section 24, for example, makes the frequency of the initial setting, which is read from a memory not shown, and the oscillation frequency of the oscillator 21 match with each other by performing control for the oscillator 21, and changes the capacitance (of the power transmission capacitor C2) such that the frequency of the initial setting matches with the series resonance frequency of the power transmission circuit 23, by performing control for the power transmission capacitor C2.

After performing step S1 of Fig. 4 or step S6 of Fig. 4 (to be described later), the control section 24 measures a reflection level RP of a power signal reflected from the power transmission circuit 23 to the power generation circuit 22 (step S2 of Fig. 4), and performs determination regarding whether or not the measured reflection level RP is more than a predetermined threshold value THR (step S3 of Fig. 4).

Then, upon obtaining a determination result that the reflection level RP measured in step S2 of Fig. 4 is not more than the predetermined threshold value THR, the control section 24 performs measurement of the reflection level RP again (step S2 of Fig. 4) while performing control for the oscillator 21 and the power transmission capacitor C2 to maintain the setting when such determination result is obtained. That is, the control section 24 infers that the series resonant frequency of the power transmission circuit 23 and the series resonance frequency of the power receiving circuit 15 match or substantially match with each other when the reflection level RP measured in step S2 of Fig. 4 is not more than the predetermined threshold value THR.

Further, upon obtaining a determination result that the reflection level RP measured in step S2 of Fig. 4 is more than the predetermined threshold value THR, the control section 24 resets the series resonance frequency of the power transmission circuit 23 to a new series resonance frequency such that the reflection level RP measured in step S2 of Fig. 4 is not more than the predetermined threshold value THR by performing control for the power transmission capacitor C2 (Step S4 of Fig. 4). That is, when the reflection level RP measured in step S2 in Fig. 4 is more than the predetermined threshold value THR, the control section 24 infers that the series resonant frequency of the power transmission circuit 23 and the series resonance frequency of the power receiving circuit 15 are relatively apart from each other.

After resetting the series resonance frequency of the power transmission circuit 23 by step S4 of Fig. 4, the control section 24 measures the phase difference between current and voltage in a power signal reflected from the power transmission circuit 23 to the power generation circuit 22 (step S5 of Fig. 4).

Then, the control section 24 performs the measurement of the reflection level RP again (Step S2 of Fig. 4) after resetting the oscillation frequency of the oscillator 21 to a new oscillation frequency such that phase difference measured in step S5 of Fig. 4 is 90° by performing control for the oscillator 21 (step S6 of Fig. 4).

Therefore, it is possible to maintain the transmission efficiency of power at a high efficiency in wireless power supply as a result of repeated performance of the control shown in Fig. 4 because, for example, even if variation occurs in the series resonance frequency of the power receiving circuit 15 associated with deformation of the extension part 32, it is possible to generate an alternating magnetic field from the power supply apparatus 20 so as to compensate for deterioration of the transmission efficiency caused by the variation of the concerned series resonance frequency.

As described so far, according to the present embodiment, it is possible to prevent, as much as possible, deterioration of the transmission efficiency of power in wireless power supply, and maintain the transmission efficiency at a high efficiency.

Note that the present embodiment will not be limited to the configuration that the extension part 32 is deformed according to the movement of the capsule endoscope 10 after the covering part 33 dissolves, but may be configured, for example, such that the extension part 32 is pre-formed so as to take on a predetermined non-linear shape (an L-shape, for example) regardless of the movement of the capsule endoscope 10. Then, according to such a configuration, it is possible to more reliably prevent deterioration of the transmission efficiency of power, which is caused depending on both orientations of the power receiving coil L1 and the power transmission coil L2.

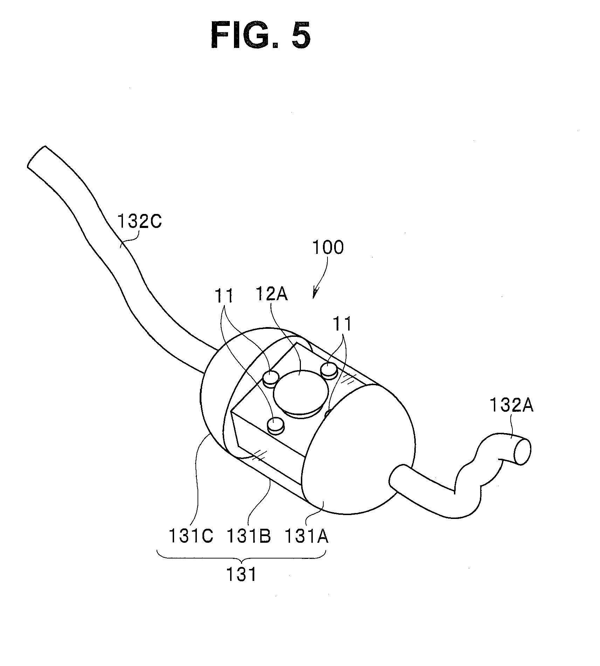

Moreover, the present embodiment is not only applicable to the capsule endoscope 10 of a front-view type having an external shape as shown in Figs. 2 and 3, but also substantially similarly applicable, for example, to a capsule endoscope 100 of a side-view type having an external shape as shown in Fig. 5. Fig. 5 is a diagram showing an example of external shape during use of a capsule endoscope according to a variant of the embodiment.

As shown in Fig. 5, the capsule endoscope 100 is configured to include: a main body part 131 having a capsule shape; an extension part 132A extending from an end part 131A of the main body part 131; and an extension part 132C extending from an end part 131C of the main body part 131.

The end part 131A of the main body part 131 is formed from a light shielding member having a dome shape. A cylindrical part 131B of the main body part 131 is formed from a cylindrical transparent member and is configured to be able to transmit illumination light emitted from the illumination section 11, and the return light incident on the objective lens 12A of the image pickup section 12, respectively. The end part 131C of the main body part 131 is formed from a light shielding member having a dome shape.

The extension parts 132A and 132C are formed from a flexible member such as of resin, respectively. Moreover, the extension parts 132A and 132C are formed to each have a shape such as an elongated tubular shape, which can contain in its internal space, at least a part of the power receiving coil L1 of the power receiving circuit 15. Note that the extension parts 132A and 132C are provided, although not shown, in a state of being folded into a predetermined shape and covered with a substance, which is similar to the water-soluble substance forming the above described covering part 33, before the capsule endoscope 10 is actually used (before being disposed in a body cavity of a subject).

Also in the capsule endoscope 100 having an external shape as described above, it is possible to achieve substantially same operational effects as in the capsule endoscope 10.

On the other hand, the present embodiment can be applied both to wireless power supply utilizing an electromagnetic induction phenomenon and to wireless power supply utilizing a magnetic field resonance phenomenon in a substantially similar manner.

The present invention will not be limited to the above described embodiment, but various changes and applications can be made, as a matter of course, within a range not departing from the spirit of the present invention.

The present application is filed based on Japanese Patent Application No. 2012-167243 filed on July 27, 2012 as the basis for claiming priority, and contents of the above described disclosure are considered to be cited in the present description, claims for patent, and drawings.

## Claims

1. A living body information acquisition system comprising a living body information acquisition apparatus configured to acquire living body information of an inside of a subject, and a power supply apparatus configured to be able to wirelessly supply power necessary for driving each part of the living body information acquisition apparatus, wherein
the living body information acquisition apparatus has a power receiving section that is capable of receiving power supplied from the power supply apparatus, and an extension part that is provided so as to extend from a main body part of the living body information acquisition apparatus, and is formed from a flexible member which can contain thereinside at least a part of the power receiving section.

2. The living body information acquisition system according to claim 1, wherein
the power receiving section is configured to resonate at a resonance frequency defined by a power receiving coil and a power receiving capacitor, and
at least a part of the power receiving coil is contained in an inside of the extension part.

3. The living body information acquisition system according to claim 2, wherein
the power supply apparatus includes:
an alternating magnetic field generation section configured to generate an alternating magnetic field corresponding to power required to drive each part of the living body information acquisition apparatus; and
a control section for performing control to cause the alternating magnetic field generation section to generate the alternating magnetic field so as to compensate for deterioration of transmission efficiency caused by variation of resonance frequency of the power receiving section associated with deformation of the extension part.

4. The living body information acquisition system according to claim 3, wherein
the alternating magnetic field generation section has a power transmission section configured to be able to generate an alternating magnetic field in accordance with power required to drive each part of the living body information acquisition apparatus by resonating at a resonance frequency defined by a power transmission coil and a power transmission capacitor, and
the control section performs control to change the resonance frequency of the power transmission section following variation of the resonance frequency of the power receiving section.

5. The living body information acquisition system according to claim 1, wherein
before the living body information acquisition apparatus is disposed in the body cavity of the subject, the extension part is provided in a state of being covered by a covering part which is formed using a water-soluble substance.

6. The living body information acquisition system according to claim 5, wherein
the extension part is configured to deform according to the movement of the living body information acquisition apparatus after the covering part dissolves.

7. The living body information acquisition system according to claim 5, wherein
the extension part is configured to take on a predetermined non-linear shape regardless of the movement of the living body information acquisition apparatus after the covering part dissolves.
